# EUROPEAN PATENT APPLICATION

(11) **EP 4 600 341 A1**
(43) Date of publication of application: **13.08.2025**
(21) Application number: 23874859.4
(22) Date of filing: 03.10.2023
(51) Int. Cl.: C12M 1/34, C12M 1/00, G06T 7/00

(54) **CULTURE DEVICE HAVING TIME-LAPSE IMAGING FUNCTION, AND CULTURE METHOD**

(30) Priority: 05.10.2022 JP 2022160883
(71) Applicant: ASTEC CO., Ltd., Fukuoka 811-2207 (JP)
(72) Inventor: INOUE, Hiromu, Kasuya-gun, Fukuoka 811-2207 (JP); FUKUDOME, Kazuki, Kasuya-gun, Fukuoka 811-2207 (JP)
(74) Representative: Winter, Brandl - Partnerschaft mbB
(86) International application number: PCT/JP2023/036069
(87) International publication number: WO 2024/075732

(57) **Abstract**

Provided is a culture device and method capable of automatically recording only one or more wells in which a cell exists within a culture container.

Provided is a culture device having a time-lapse shooting function of performing time-lapse shooting, wherein a cell is placed to be cultivated in each well of a culture container including at least one or more wells. The culture device comprises: a shooting means shooting an image of each well of the culture container in a predetermined time-lapse shooting cycle; a detection means detecting presence/absence of a cell within each well of the culture container; and an image recording means recording an image of a well wherein the presence of the cell is detected by the detection means among images of each well shot by the shooting means.

## Description

### TECHNICAL FIELD

The present invention relates to a culture device and a culturing method, each of which has a time-lapse shooting function and performs time-lapse shooting while culturing cells, such as embryos.

### BACKGROUND ART

Culture devices having a time-lapse shooting function of performing time-lapse shooting while culturing cells, such as embryos are known. (For example, see References 1 to 3.)
For example, in a certain culture device having the time-lapse shooting function, a plurality of fertilized eggs (embryos) extracted from one patient are placed into a plurality of wells divided within one culture container to start cultivation thereof. The time-lapse shooting is performed in a constant cycle until the embryos develop to be implantable (for three to seven days), and the shot image data is stored onto a recording medium.

And then, the images time-lapse shot of the plurality of embryos in growth process are morphologically evaluated to judge whether the embryos are good or bad. And then, the embryos are transferred, cryopreserved, or discarded.
In general, the evaluation of the embryo images is performed by means of image displaying software for assisting the embryo evaluation. The image displaying software performs display in the order of time scales, and the user thereof confirms the embryo images in growth process to perform judgment thereof.

### PRIOR ART DOCUMENTS

### PATENT DOCUMENTS

Reference 1: Japanese patent application Laid-open No. 2009-539387
Reference 2: Japanese patent application Laid-open No. 2017-529844
Reference 3: Japanese patent application Laid-open No. 2016-123366

### SUMMARY OF THE INVENTION

### PROBLEMS TO BE SOLVED BY THE INVENTION

By the way in some cases, the cultivation of a part of the embryos may be ended in order to perform cryopreservation, transplant, and so on. Otherwise, the part of the embryos may be temporarily taken out from the device in order to perform microscope observation, and medium exchange thereof.
In the time-lapse shooting, a well wherein an embryo within the culture container is placed is specified in advance at dish-in when cultivation starts, and the specified well is periodically shot. Even if an embryo is taken out from the specified well, the specified well without the embryo is also shot to be stored onto a recording medium. In this way, recording medium usage may increase more than necessary, thereby causing the shortage of the recording medium.

When the image displaying software performs display in the order of time scales, or displays moving pictures frame-by-frame, unnecessary images without an embryo are also displayed. In this way, the display may become difficult to see, and the efficiency of the evaluation may tend to decrease.

In view of the above, an object according to the present invention is provide a culture device and a culture method, each of which having time-lapse shooting function and being capable of automatically recording only one or more wells in which a cell exists within a culture container.

### MEANS FOR SOLVING THE PROBLEM

Provided is a culture device of the present invention having a time-lapse shooting function of performing time-lapse shooting, wherein a cell is placed to be cultivated in each well of a culture container including at least one or more wells, the culture device comprising: an shooting means shooting an image of each well of the culture container in a predetermined time-lapse shooting cycle; a detection means detecting presence/absence of a cell within each well of the culture container; and an image recording means recording an image of a well wherein the presence of the cell is detected by the detection means among images of each well shot by the shooting means.

According to the culture device having the time-lapse shooting function according to the present invention, every well of the culture container is shot in the predetermined time-lapse cycle, and the presence/absence of every cell is detected. Furthermore, only well images wherein the presence of the cell is detected are recoded onto the image recording means.

It is preferable that the detection means detects the presence/absence of the cell by analyzing the images of each well shot by the shooting means.
With this arrangement, the shot images of each well are analyzed, and the presence of the cell is detected in some cases. As a result of this analyzing, only the well images wherein the presence of a cell is detected are recorded onto the image recording means.

It is preferable that the detection means detects the presence/absence of the cell in the time-lapse shooting cycle.
With this arrangement, whenever the presence/absence of the cell is detected in the predetermined time-lapse shooting cycle. As a result of this detection, only the well images wherein the presence of a cell is detected are recorded onto the image recording means.

It is preferable that the culture device having the time-lapse shooting function further comprises a notification means performing notification when a current detection result detected by the detection means differs from a previous detection result.
With this arrangement, since notification is performed when a current detection result detected by the detection means differs from a previous detection, a user can confirm the detection result.

It is preferable that the culture device having the time-lapse shooting function further comprises a history recording means recording, in the time-lapse shooting cycle, histories regarding the presence/absence of the cell of all wells of the culture container. With this arrangement, in the time-lapse shooting cycle, histories regarding the presence/absence of the cell of all wells of the culture container are recorded by the history recording means. Accordingly, based on the histories regarding the presence/absence of the cell of all wells of the culture container, display by means of an image array in timeline and/or management of the histories can be performed.

Provided is a culture method of the present invention wherein a cell is placed to be cultivated in each well of a culture container including at least one or more wells, the culture method comprising: shooting an image of each well of the culture container in a predetermined time-lapse shooting cycle; detecting presence/absence of a cell within each well of the culture container; and recording an image of a well wherein the presence of the cell is detected among shot images of each well.

According to the culturing method according to the present invention, an image of each well of the culture container is shot in a predetermined time-lapse shooting cycle, the presence/absence of a cell within each well of the culture container is detected, and only an image of a well wherein the presence of the cell is detected among shot images of each well is recorded.

### EFFECTS OF THE INVENTION

According to the present invention, only the well images wherein a cell exists can be automatically recorded, it is unnecessary to register at dish-in beforehand wells wherein a cell exists and requires shooting thereof. In this way, necessary work can be reduced.
It is also capable of reducing the amount to be used for recording images of the recording medium.
Since unnecessary well images wherein a cell does not exist are not recorded, flexibility with respect to shooting cycle and/or schedule setting can be increased, thereby enabling culture according to image observation with higher precision. Since there is no unnecessary well image wherein a cell does not exist, evaluation with high efficiency can be made when displaying recorded images.

### BRIEF DESCRIPTION OF THE DRAWINGS

Fig. 1 shows a schematic structure of a culture device having a time-lapse shooting function in an Embodiment according to the present invention.
Fig. 2 shows an enlarged structure of the culture container of Fig. 1.
Fig. 3 is a functional block diagram of the culture device having the time-lapse shooting function of Fig. 1.
Fig. 4 shows an example of a detection process with respect to the presence/absence of a cell.
Fig. 5 is a flaw chart showing a time-lapse shooting process according to the culture device.
Fig. 6 (A) shows an example of live images of all wells (prior art).
Fig. 6 (B) shows an example of live images of all wells (present invention).
Fig. 7 (A) shows an example of time-line display of a plurality of wells (prior art).
Fig. 7 (B) shows an example of time-line display of a plurality of wells (present invention).
Fig. 8 shows a comparative example displaying moving pictures according to time-lapse shooting frame-by-frame.
Fig. 9 shows an example displaying abnormal notification.

### DETAILED DESCRIPTION OF THE EMBODIMENTS

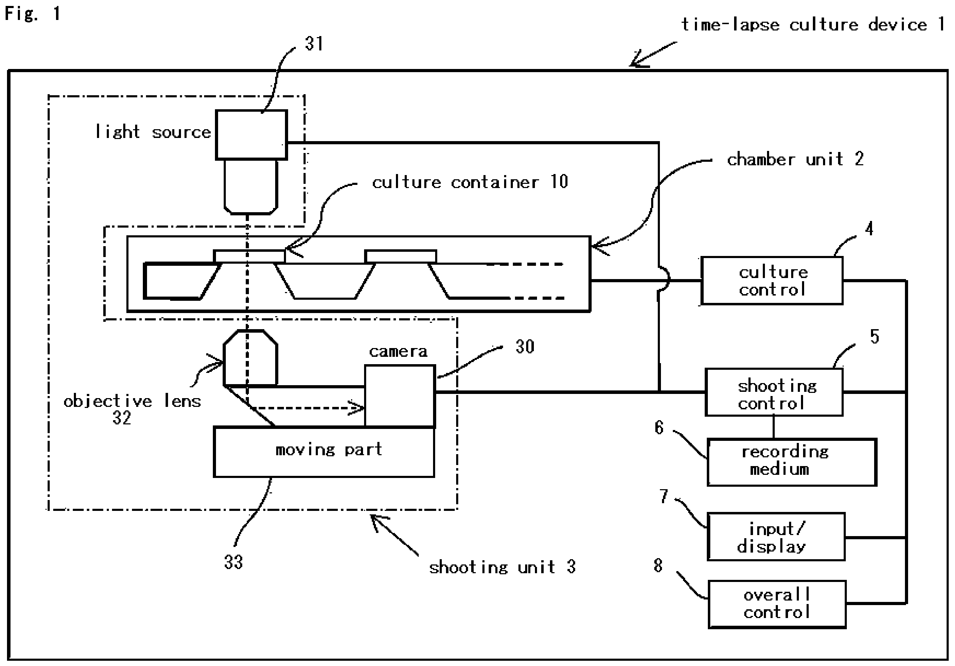

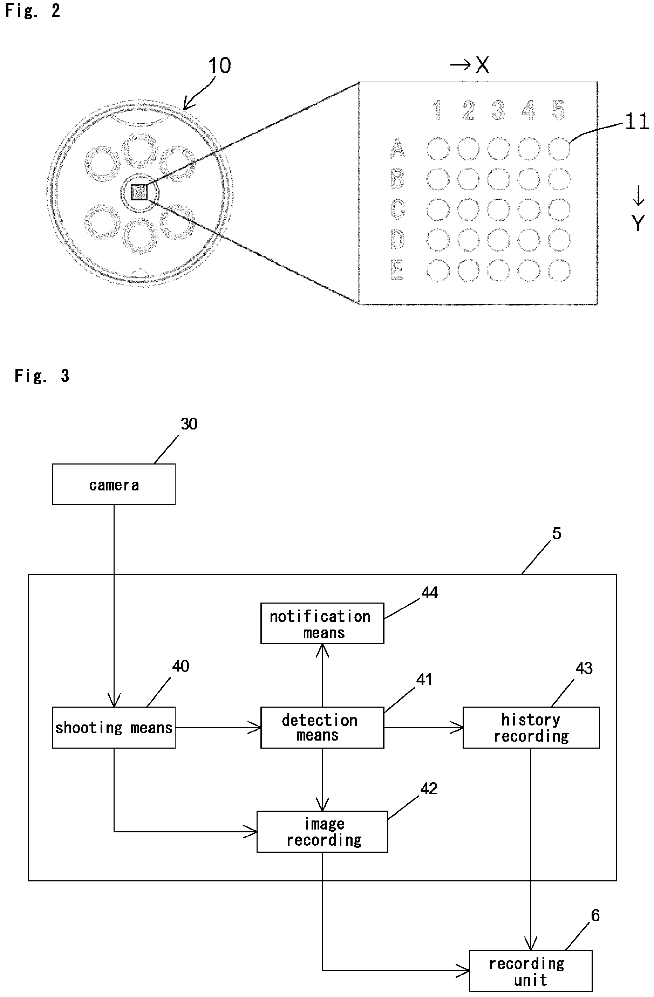

Fig. 1 shows a schematic structure of a culture device having a time-lapse shooting function in an Embodiment according to the present invention, Fig. 2 shows an enlarged culture container of Fig. 1, and Fig. 3 is a functional block diagram of the culture device having the time-lapse shooting function of Fig. 1.

As shown in Fig. 1, a culture device 1 having a time-lapse shooting function in an Embodiment according to the present invention, which hereinafter may be simply called as a "culture device," includes: a chamber unit 2; and a culture container 10 within the chamber unit 2. The culture container 10 includes at least one or more wells 11 (See, Fig. 2.). Cells are placed to be cultivated in the respective wells 11. And, an shooting unit 3 of the culture device 1 performs time-lapse shooting. Herein, the time-lapse shooting means a shooting method including: shooting a plurality of still images at a fixed time interval (in a time-lapse cycle); joining the plurality of still images to create moving pictures; and so on.

In addition, the culture device 1 includes: a culture control unit 4 which controls the chamber unit 2; an shooting control unit 5 which controls an shooting unit 3; a recording unit 6 which stores images shot by the shooting unit 3; an input/display unit 7 which enters instructions to the culture device 1 and displays images; and an overall control unit 8 which controls the entire culture device 1.

The chamber unit 2 maintains environment required for cultivation of cells, such as temperature, concentration of CO₂ gas and/or O₂ gas, or the like. One or more culture containers 10 can be stored within the chamber unit 2. A heater, a CO₂ gas flow path, an O₂ gas flow path, or the like (Not shown in Figs.) are connected there-between within the chamber unit 2.
The culture control unit 4 controls the heater, the CO₂ gas flow path, the O₂ gas flow path, or the like so that the temperature and concentration of CO₂ gas and/or O₂ gas are constant.
Herein in Fig. 1, two culture containers 10 are shown within the one chamber unit 2. However alternatively, the chamber unit may be composed of multiple chambers which are divided for one or more culture containers.

The shooting unit 3 is composed including: a camera 30; a light source 31; an objective lens 32; a moving part 33; or the like. In the shooting unit 3, light irradiated by the light source 31 is applied to the cell within the culture container 10, the objective lens 32 receives penetration light and diffraction light thereby, and images enlarged by the objective lens 32 are inputted into the camera 30.
The moving part 33 makes both of the camera 30 and the light source 31 integrally move in directions (X and Y directions of Fig. 2) wherein the wells of the culture container 10 are arranged.
In addition, the moving part 33 makes the objective lens 32 in the Z-axis (an optical axis) direction so as to enable to shoot the cells within the plurality of culture containers 10.
Alternatively, a plurality of sets of the camera 30 and the light source 31 may also be provided and arranged for every culture container 10 and/or every chamber unit 2. In another Embodiment, the camera 30 and the light source 31 may be fixed. The chamber unit 2 may be constituted integrally with another moving unit (Not shown.). In this way, the culture container 10 may be constituted so that the culture container 10 is movable in the X, Y, and Z directions.
In further another Embodiment, the camera 30 and the light source 31 may be fixed. And, both of the chamber unit 2 and the culture container 10 may be made movable.

The shooting control unit 5 controls: optical matters of the shooting unit 3 upon shooting images; positioning; time-lapse shooting conditions; recording images; or the like.
As shown in Fig. 3, the shooting control unit 5 includes:
an shooting means 40 shooting images of every well 11 of the culture container 10 according to a predetermined time-lapse shooting cycle;
a detection means 41 detecting presence/absence of a cell for every well 11 of the culture container 10; and
an image recording means 42 recording images of the well 11 wherein the presence of the cell is detected by the detection means 41 among images shot for every well 11 by the shooting means 40.

By means of the shooting unit 3, the shooting means 40 shoots images of the respective wells 11 of the culture container 10 according to a predetermined time-lapse cycle. The time-lapse cycle may be set up arbitrarily (for example, from several minutes to several hours).
The shooting unit 3 may be composed of a microscope camera including: an objective lens (for example, magnification of about 10 times); CCD (Charge-Coupled Device) and/or CMOS (Complementary Metal Oxide Semiconductor) image sensors; or the like.

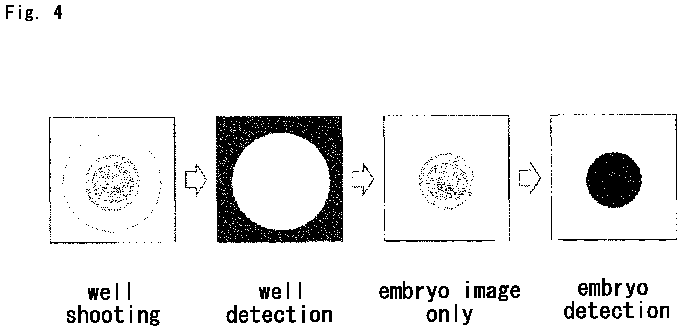

The detection means 41 detects the presence/absence of a cell by analyzing images shot for every well by the shooting means 40. Detection of the presence/absence of the cell is performed in a unit of one well. Fig. 4 shows an example of a detection process with respect to the presence/absence of a cell.
In the example shown in Fig. 4, the detection means 41 detects a well based on the well images of the well shot by the shooting means 40 to produce an embryo only image, and detects the presence/absence of the cell in accordance with the produced embryo only image.
The detection of the presence/absence of the cell in accordance with the produced embryo only image can be performed according to methods by means of image processing, artificial intelligence (machine learning and/or deep learning), or the like.

For example, there is a method wherein: objects are extracted by labeling images; and the presence/absence of the embryo is detected in accordance with the extracted objects.
The embryo images have the following characteristics. The presence/absence of the embryo can be detected focusing on feature wherein one or more of the following characteristics are combined with each other based on the extracted objects.

The shape thereof is circular;
the size thereof is from 100 micrometers to 200 micrometers;
the outline thereof has an annular transparent body (almost uniform brightness range in the image); and
the inside thereof has a structure (texture).

Alternatively instead of the detection in accordance with the object extraction, it may be adopted to a method of performing detection by means of deep learning or the like based on image characteristics, such as brightness characteristics of the entire image, extraction of feature points, model comparison, or the like.

The image recording means 42 records onto the recording unit 6 only the image of the well wherein the presence of the cell is detected by the detection means 41 among images for every well shot by the shooting means 40.
The recording unit 6 is a recording medium, for example, a magnetic disk (such as a hard disk drive (HDD) and a flexible disk (FD)), an optical disk (such as a compact disk (CD) and an DVD), a magneto-optical disk (such as an MO), a solid state drive (SSD), a flash memory (such as a memory card and a USB memory), a magnetic tape, or the like.
It is also possible to use another recording medium (such as network attached storage (NAS), cloud storage, or the like) as the recording unit 6.

The timing wherein the detection means 41 detects the presence/absence of the cell may be when the time-lapse shooting starts, during the time-lapse shooting, or both of them. It is preferable to detect the presence/absence of a cell in every time-lapse cycle. With this arrangement, every time when an image is shot in a predetermined time-lapse cycle, the presence/absence of a cell is detected. Only well images wherein the presence of a cell is detected are recorded onto the image recording means 42. **In** this way, only the minimum required well images are recorded onto the image recording means 42.

The input/display unit 7 includes a display device provided with a touch panel, or the like.
The input/display unit 7 gives entered instructions from a user to the culture control unit 4 and the shooting control unit 5. In addition, the input/display unit 7 displays images shot by the camera 30, images read from the recording unit 6, or the like.

The overall control unit 8 controls communications between the respective blocks of the device, and all elements provided with the device.
The overall control unit 8 also includes an interface for the exterior of the device. Software for performing observation and evaluation of embryos may be equipped with the overall control unit 8.
With this arrangement, the overall control unit 8 can assist user's cultivation evaluation business effectively, whereby the overall control unit 8 makes the input/display unit 7 arrange images clearly and display analysis results.

Next, a time-lapse shooting method by means of the culture device 1 composed as mentioned in the above will now be explained.

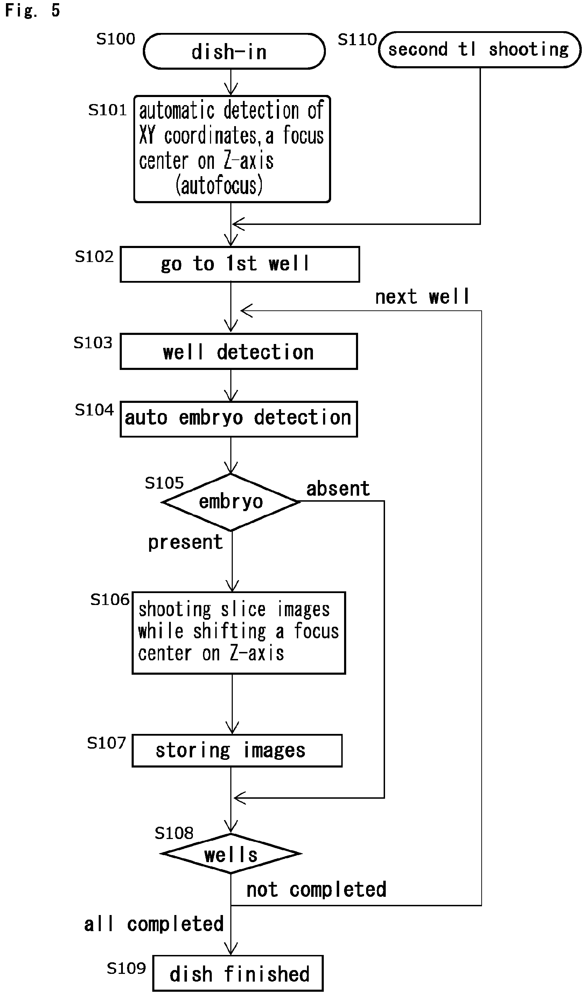

Fig. 5 is a flaw chart showing a time-lapse shooting process according to the culture device 1.

At the step S100, a dish in process wherein the culture container (dish) 10 goes into the inside of the culture device 1 is started.
At the step S101, automatic detection of XY coordinates and a focus center on Z-axis (autofocus) with respect to all wells 11 is performed in accordance with marks or the like provided within the culture container 10.
At the step S102, the shooting means 40 moves the shooting unit 3 to the first well 11.
At the step S103, the shooting means 40 shoots the first well 11 by means of the shooting unit 3, and the detection means 41 detects the first well 11 based on the image shot by the shooting means 40.
At the step S104, the detection means 41 automatically detects the presence/absence of an embryo within the first well 11.
At the step S105, the detection means 41 judges the presence/absence of the embryo. If there is no embryo within the first well 11, process goes to the step S108.
At the step S106, if there is an embryo within the first well 11, the shooting means 40 shoots a plurality of slice images while shifting a focus center on Z-axis back and forth from an original focus center on Z-axis. On the contrary, if the slice images are not needed, it is sufficient to shoot only an image on the original focus center on Z-axis.
At the step S107, the image recording means 42 records the shot images of the first well 11 onto the recording unit 6.
At the step S108, it is judged whether or not shooting all of the wells 11 has been completed. If the shooting has not been completed, process goes back to the step S103 in order to shoot the next well 11.
If the shooting all of the wells 11 has been completed, process is ended at the step S109.
In the time-lapse shooting in second time-lapse cycle onwards, process is started from the step S102.

In this Example, it is adopted a method of detecting the well 11 firstly, and then detecting a cell. Alternatively, it may be adopted another method of searching directly the cell in accordance with the regularity of well arrangement or the like to detect the presence/absence of the cell without detecting the well 11 itself.

In also this Example, X, Y, and Z coordinates of all of the wells 11 are detected at the step S101. A plurality of divisional images each of which includes a plurality of wells may be shot with respect to the entire culture container 10 to be tiled. Otherwise, all of the wells 11 may be widely shot as one large image by means of a camera with high resolution, and the one large image are divided into divisional images for extracting the respective well image to detect the presence/absence of a cell therein.

As shown in Fig. 3, in the culture device 1 in this Embodiment, the shooting control unit 5 may be constituted including:
a history recording means 43 recording, in the time-lapse shooting cycle, histories regarding the presence/absence of the cell of all wells 11 of the culture container 10; and
a notification means 44 performing notification when a current detection result detected by the detection means 41 differs from a previous detection result.

The history recording means 43 records, in the time-lapse shooting cycle, histories regarding the presence/absence of the cell of all wells 11 of the culture container 10 based on a detection result by the detection means 41.
With this arrangement, the histories regarding the presence/absence of the cell of all wells 11 of the culture container 10 are recorded by the history recording means 43 in every time-lapse photography cycle.
This arrangement enables image arrangement according to a time-line display, and/or history management based on the histories regarding the presence/absence of the cell of all wells 11 of the culture container 10 in every time-lapse photography cycle.

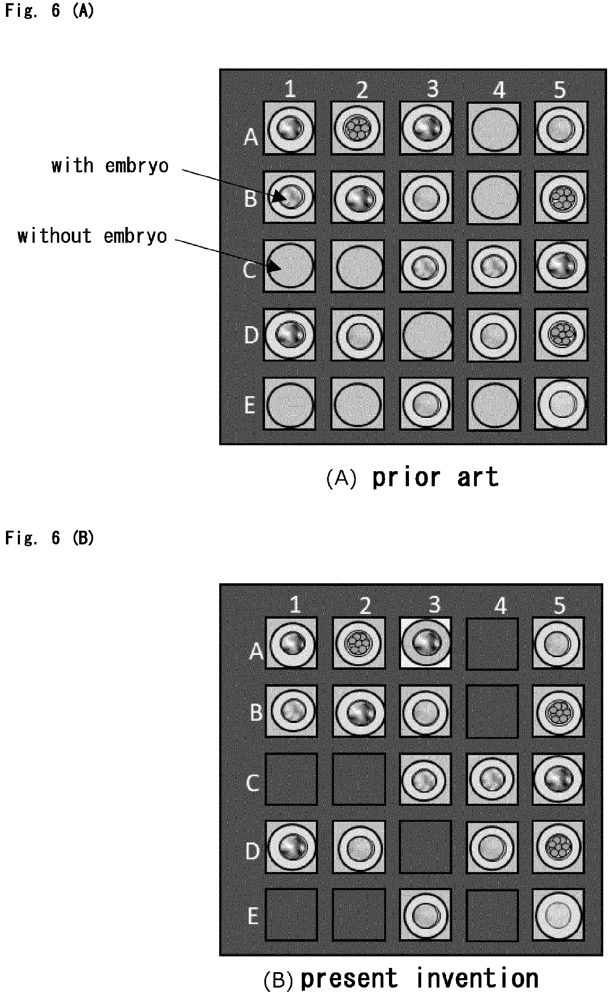

Fig. 6 (A) shows an example of live images of all wells (prior art), and Fig. 6 (B) shows an example of live images of all wells (present invention). As shown in Fig. 6 (A) in prior art, images of all the wells 11 within a culture container 10 are recorded, and the images of all the wells 11 are displayed regardless of the presence/absence of an embryo within the respective well 11. On the contrary according to the present invention, only well images wherein the presence of a cell is detected among all the wells 11 within the culture container 10 are recorded onto the image recording means 6, and the histories regarding the presence/absence of an embryo of all wells 11 are recorded by the history recording means 43.
As shown in Fig. 6 (B) based on the histories regarding the presence/absence of an embryo, it is possible to display only the images of the well 11 with an embryo, while the wells 11 without an embryo are not shown. Accordingly, upon performing comparative observing and/or evaluating embryos by means of observation and/or evaluation software with respect to images recorded onto the recording unit 6, it is easy to see the image at a glance to understand conditions and/or progress, thereby enabling to perform cultivation observation and/or evaluation thereof with high efficiency.

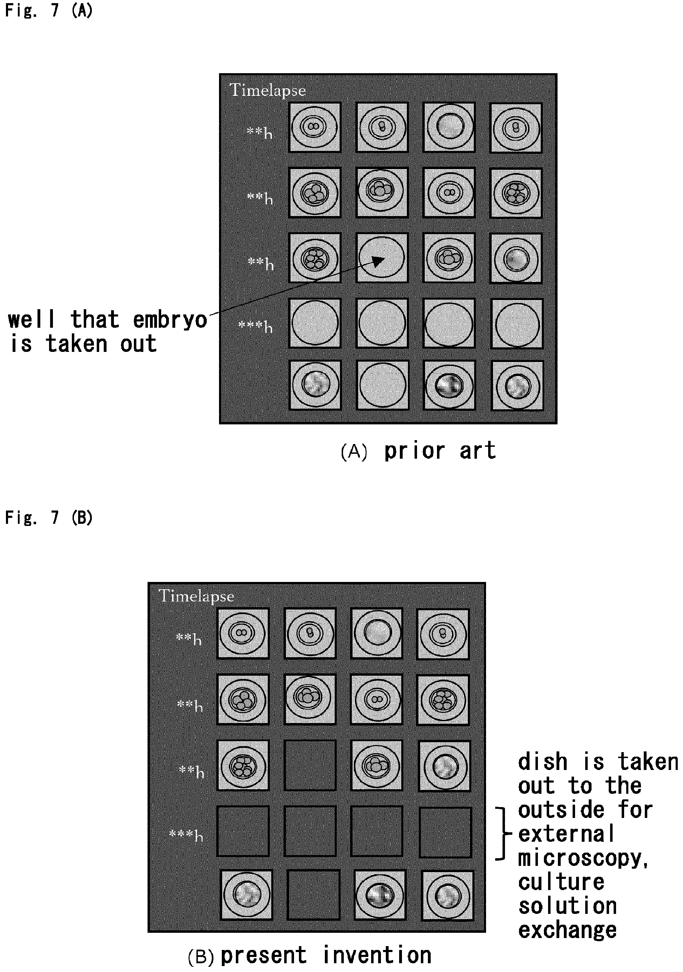

Fig. 7 (A) shows an example of time-line display of a plurality of wells (prior art), and Fig. 7 (B) shows an example of time-line display of a plurality of wells (present invention).
In Fig. 7, a plurality of wells are arranged in a lateral direction, and well images in every time-lapse cycle in a vertical direction are arranged to be displayed thereon. In some stages of cell culture, an embryo may be taken out from the well 11 in order to perform cryopreservation and/or transplant thereof in the middle of the cultivation. Furthermore, in another case, the entire culture container (dish) 10 itself may be taken out in order to perform external microscopy and/or culture solution exchange thereof. In this case according to prior art as shown in Fig. 7 (A), all the wells 11 including the wells 11 wherein an embryo has been taken out therefrom are also be displayed. On the other hand, according to the present invention as shown in Fig. 7 (B), the wells 11 wherein an embryo has been taken out therefrom in order to perform cryopreservation and/or transplant thereof in the middle of the cultivation are not displayed based on the histories regarding the presence/absence of an embryo recorded by the history recording means 43.
Furthermore, when the entire culture container (dish) 10 itself may be taken out to the outside in order to perform external microscopy and/or culture solution exchange thereof, display thereof is not performed. In a case wherein the culture container 10 is rearranged, only the wells 11 with an embryo are displayed again.

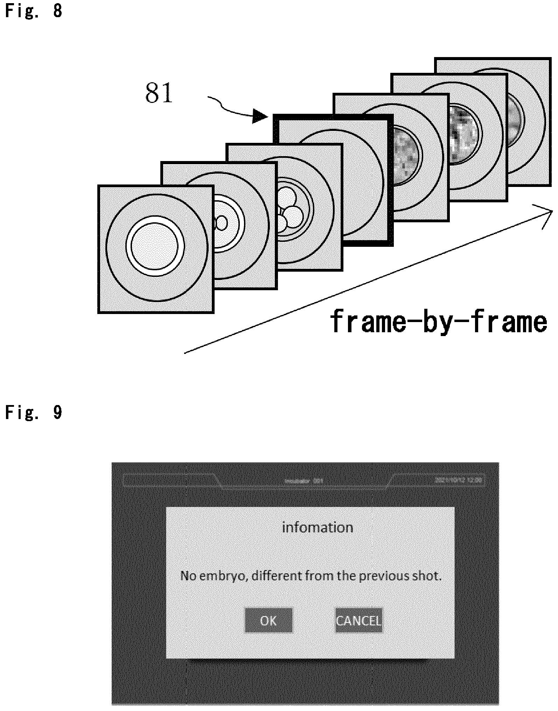

Fig. 8 shows a comparative example displaying moving pictures according to time-lapse shooting frame-by-frame.
As shown in Fig. 8 according to prior art, the time-lapse image data of a predetermined embryo are read to be displayed continuously from a storage unit, an image 81 of a well without a cell is also displayed in a mixed manner.
According to prior art upon displaying the moving pictures, one or more images without an embryo suddenly appears, the results thereof are discontinuous and hard to see.
On the other hand, in the culture device 1 having a time-lapse shooting function in this Embodiment, the one or more images without the embryo are excluded. In this way, the results of moving picture which are continuous and easy to see can be obtained. That is, in culture device 1 having the time-lapse shooting function in this Embodiment, by displaying time-lapse images continuously, the growth state of a cell can be continuously visualized, and the process of change and/or growth thereof can be understood at ease.

The notification means 44 performing notification when a current detection result detected by the detection means 41 differs from a previous detection result. The notification means 44 may include: a ring of a notice buzzer; a light of a notice lamp, an abnormal notification display; or the like. Fig. 9 shows an example displaying abnormal notification. The notification means 44 performing notification when the current detection result detected by the detection means 41 differs from the previous detection result. For example as shown in Fig. 9, the notification means 44 performs the abnormal notification by displaying a dialog on the display so as to call for the attention of a user of the culture device 1, thereby confirming whether or not shooting is needed. Herein, shooting operation of the respective well 11 is set up to be in a waiting state. The user may select the "OK" bottom when the presence/absence of an embryo can be confirmed due to cryopreservation and/or transplant thereof. On the other hand when it seems that judgment may include an error, the user may select the "CANCEL" bottom to instruct to shoot another image. This Embodiment enables such an operation. In another Embodiment, it is possible to inform to a user of detection result of the presence/absence of a cell with information with respect to confirmation of shooting necessity, an abnormal state regarding dish installation, or the like.

As mentioned above in the culture device 1 in this Embodiment, only the images of the wells 11 wherein a cell exists can be automatically recorded for every well 11 of the culture container 10, it is capable of reducing the amount used for recording images onto the recording medium 6.
Since unnecessary images of the well 11 wherein a cell does not exist are not recorded, flexibility with respect to shooting cycle and/or schedule setting can be increased, thereby enabling culture according to image observation with higher precision. Since there is no unnecessary well image wherein a cell does not exist, evaluation with high efficiency can be made.

### INDUSTRIAL APPLICABILITY

The present invention is effective as a culture device and a culture method, each of which has a time-lapse shooting function of performing time-lapse shooting while culturing cells, such as embryos.

### EXPLANATION OF REFERENCE NUMERALS

1: Culture Device Having Time-lapse Shooting Function
2: Chamber Unit
3: Shooting Unit
4: Culture Control Unit
5: Shooting Control Unit
6: Recording Unit
7: Input/display Unit
8: Overall Control Unit
10: Culture Container
11: Well
30: Camera
31: Light Source
32: Objective Lens
33: Moving Part
40: Shooting means
41: Detection Means
42: Image Recording Means
43: History Recording Means
44: Notification Means

## Claims

1. A culture device having a time-lapse shooting function of performing time-lapse shooting, wherein a cell is placed to be cultivated in each well of a culture container including at least one or more wells, the culture device comprising:
a shooting means shooting an image of each well of the culture container in a predetermined time-lapse shooting cycle;
a detection means detecting presence/absence of a cell within each well of the culture container; and
an image recording means recording an image of a well wherein the presence of the cell is detected by the detection means among images of each well shot by the shooting means.

2. The culture device having the time-lapse shooting function as defined in claim 1, wherein the detection means detects the presence/absence of the cell by analyzing the images of each well shot by the shooting means.

3. The culture device having the time-lapse shooting function as defined in claim 1 or 2, wherein the detection means detects the presence/absence of the cell in the time-lapse shooting cycle.

4. The culture device having the time-lapse shooting function as defined in claim 1 or 2, further comprising a notification means performing notification when a current detection result detected by the detection means differs from a previous detection result.

5. The culture device having the time-lapse shooting function as defined in claim 1 or 2, further comprising a history recording means recording, in the time-lapse shooting cycle, histories regarding the presence/absence of the cell of all wells of the culture container.

6. A culture method wherein a cell is placed to be cultivated in each well of a culture container including at least one or more wells, the culture method comprising:
shooting an image of each well of the culture container in a predetermined time-lapse shooting cycle;
detecting presence/absence of a cell within each well of the culture container; and
recording an image of a well wherein the presence of the cell is detected among shot images of each well.
